# EUROPEAN PATENT SPECIFICATION

(11) **EP 0 951 269 B1**
(45) Date of publication and mention of the grant of the patent: **16.08.2001**
(21) Application number: 96944466.0
(22) Date of filing: 18.12.1996
(51) Int. Cl.: A61K 7/021, A61K 7/02, A61K 7/42

(54) **STABLE HIGH SPF FOUNDATION**
STABILES MAKE-UP MIT HOHEM SONNENSCHUTZFAKTOR
FOND DE TEINT STABLE A FPS ELEVE

(43) Date of publication of application: 27.10.1999
(73) Proprietor: THE PROCTER & GAMBLE COMPANY, Cincinnati, Ohio 45202 (US)
(72) Inventor: MASUDA, Hisatoshi, Moriyama-shi, Shiga 520-21 (JP)
(74) Representative: Woof, Victoria
(86) International application number: US9620215
(87) International publication number: WO9826752

(56) References cited:
- EP-A- 0 456 459
- EP-A- 0 600 445
- WO-A-96/33689
- CHEMICAL ABSTRACTS, vol. 124, no. 4, 22 January 1996 Columbus, Ohio, US; abstract no. 33016, OKUDA, HARUO ET AL: "Titanium dioxide aqueous dispersions for UV-shielding agents" XP002039358 cited in the application & JP 07 247 119 A (ISHIHARA SANGYO KAISHA, JAPAN)

## Description

### Field of the invention

This invention relates to stable high SPF (Sun Protection Factor) value foundations and a method for preparing them.

### Background

One of the major purposes of skin cosmetics is to improve the outward, especially facial, appearance. Typically foundations are used to enhance features, or mask perceived imperfections in them. As foundations are typically applied prior to other color cosmetics, they provide a uniform base of color and coverage which improve the overall appearance of make-up. Characteristics considered by consumers when choosing a foundation fall into three general areas: look (or appearance both upon application and after wear), feel (e.g., ease of application and the feel of the "made up" area), and wear (resistance to water, oil, abrasion, etc.). Such foundations are generally available in the form of liquid, semiliquid or cream suspensions, emulsions, gels, as well as pressed powders or anhydrous oil and wax compositions.

Recently, due to the development of silicone oil, water-in-oil type foundations have been focused on.

A film forming water-in-oil emulsion foundation comprising a water compatible film forming polymer and plasticizing solvent in the aqueous phase is disclosed in PCT patent application No.US 96/4302 (Canter, Barforld and Hofrichter).

In order to protect skin from ultraviolet, titanium oxide is commonly used as an ultraviolet-reflecting agent. However, titanium oxide having a large particle size causes an undesirable high coverage look to skin.

An ultra fine particle size titanium dioxide is known to be very effective to obtain a cosmetic with adequate coverage effect and high SPF value. However, if an ultra fine titanium oxide is simply mixed with an emulsion foundation, the ultra fine titanium dioxide agglomerates. As a result, only a foundation having a high coverage effect and low SPF value can be obtained. Also, if a foundation comprises a high level of a ultra fine titanium oxide, sufficient spreadability and suitable feel during application cannot be obtained.

In order to solve the above problem, a water dispersion or an oil dispersion of a ultra fine titanium dioxide with dispersing agent is available for cosmetics in the market. A foundation comprising a water dispersion or oil dispersion of an ultra fine titanium dioxide can obtain adequate coverage effect and good SPF value with a minimum amount of the ultra fine titanium dioxide. Unfortunately, current water-in-oil foundations comprising a water dispersion or oil dispersion of an ultra fine titanium dioxide have certain disadvantages, including:
(1) A water-in-oil foundation comprising an oil dispersion of an ultra fine titanium dioxide causes tight feel to the user. This tight feel sensation results from the ultra fine titanium dioxide being out of phase of the emulsion, although the ultra fine titanium dioxide is stably dispersed in the oil phase of the foundation.
(2) A water-in-oil foundation comprising a water dispersion of an ultra fine titanium dioxide destroys not only the balance of the stable emulsion but also results in agglomeration of the ultra fine titanium dioxide (although the agglorrerated titanium dioxide remains stably dispersed). This destruction and agglomeration are due to the fact that the water dispersion of an ultra fine titanium dioxide comprises a hydrophilic dispersant. However, the foundation comprising the water dispersion of an ultra fine titanium dioxide does not cause tight feel.

PCT Patent Publication No. WO 94/15580 (publication date: July 21,1994/inventor: Jorgensen) discloses a tinted oil-in-water emulsion comprising a finely divided pigment of which the surface has been treated and a carboxylic acid polymer. Japanese Patent Laid-Open No. 1-180819 (publication date: July 18,1989/inventors: Kawai, Mouri and Okuda) discloses a cosmetic comprising a water dispersion of an ultra fine titanium dioxide having a specific narrow range of particle size and water soluble high molecular substance. Yasuhiro Ohara and Shu Sakuyama, Fragrance Journal Vol.12 page 14-16 (1993) discloses that a water-soluble polymer may be used for stabilizing dispersions.

WO 96/33689 discloses water in oil emulsions with titanium dioxide and nonionic surfactant without specific concentrations or ranges thereof.

A water-in-oil emulsion foundation comprising a water dispersion of an ultra fine titanium dioxide, however, still destroys the balance of the stable emulsion and agglomerates the ultra fine titanium oxide. Consequently, there is a need for a stable water-in-oil emulsion foundation comprising a water dispersion of an ultra fine titanium dioxide containing a dispersant, which does not destroy the balance of the stable emulsion and does not agglomerate the ultra fine titanium dioxide.

### SUMMARY

The present invention relates to a stable water-in-oil emulsion foundation wherein the aqueous internal phase comprises: a) from about 0.1% to about 10 % by weight of the foundation, of a water soluble or dispersible polymer; b) from about 0.5% to about 30 % by weight of the foundation, of a plasticizing solvent and c) from about 1% to about 30% by weight of the foundation, of an ultra fine titanium dioxide-water dispersion. The water dispersion comprises from about 0.2% to about 18% by weight of the foundation of an ultra fine titanium dioxide, and from about 0.002% to about 7.2% by weight of the foundation of a nonionic surfactant.

The present invention also relates to a process for preparing a stable water-in-oil emulsion foundation comprising the steps of a) mixing oil-soluble components to obtain an oil mixture, b) mixing water-soluble components and a water soluble or dispersible polymer to obtain an aqueous mixture, c) mixing the aqueous mixture and the oil mixture together to obtain an emulsion, and d) mixing the emulsion and an ultra fine titanium dioxide-water dispersion together, wherein the water dispersion comprises an ultra fine titanium dioxide and a nonionic surfactant.

### DETAILED DESCRIPTION

All amounts specified herein are by "weight percent", unless otherwise specifically stated.

As used hereinafter, the term "foundation" refers to a liquid or semi-liquid skin cosmetic which includes, but is not limited to, lotions, creams, gels, pastes, and the like. Typically the foundation is used over a large area of the skin, such as over the face, to provide a particular look. Such a composition is typically in the form of a water-in-oil emulsion.

The foundation of the present invention comprises a water soluble or dispersible polymer, a plasticizing solvent and an ultra fine titanium dioxide-water dispersion in the aqueous (internal) phase of the emulsion. This minimizes the unpleasant tacky sensation characteristic of polymers on the user's hands and fingers during application of the cosmetic. This also minimizes tight feel related to the ultra fine titanium dioxide. The combination of aqueous phase polymer and solvent is chosen to provide proper evaporation rate and polymer solvation for extension of the workability of the foundation and delay of any perceived onset of tackiness until after application is complete.

Preferably, a pigment other than ultra fine titanium dioxide is present in the oil phase of a water-in-oil emulsion, to provide even coverage during initial application of the foundation.

Such a foundation satisfies the need for a stable water-in-oil emulsion foundation comprising a water dispersion of an ultra fine titanium dioxide, which does not destroy the balance of the stable emulsion and does not agglomerate the ultra fine titanium dioxide

Another possible benefit of the foundation of the present invention is having an improved SPF value.

A further possible benefit of the foundation of the present invention is (i) suitable feel and appearance during application, (ii) excellent wear and appearance benefits after application, (iii) a flexible, light feel that resembles other foundations, and (iv) being easily removed with soap and water.

### A. Emulsion

It is well understood by the skilled artisan that a water-in-oil emulsion has hydrophylic or aqueous material dispersed in hydrophobic or "oil"-like material. Thus the internal or dispersed phase is aqueous or "water"-like in nature and is called the "aqueous phase." The external or continuous phase is hydrophobic, and is called the "oil phase."

The aqueous phase of the emulsion comprises a water soluble or dispersible polymer, a plasticizing solvent, water soluble additives such as a moisturizer, an electrolyte and water. Preferably water can comprise up to about 60% by weight of the foundation. Preferably, the water is present in an amount of from about 10 % to about 50% by weight of the foundation.

The oil (external) phase may comprise branched paraffins, hydrocarbons, esters, ethers, silicones and the like. Preferably the oil phase is comprised of volatile material and contains no "oils" as defined by the C.T.F.A. (The Cosmetic, Toiletry, and Fragrance Association) Cosmetic Ingredient Handbook, 3rd Ed., Cosmetic and Fragrance Assn., Inc., Washington D.C. (1982)., incorporated herein by reference. More preferably, the oil phase comprises silicones, more preferably up to about 90% of the oil phase is volatile silicones, non-volatile silicones and mixtures thereof. Still more preferably, these silicones are chosen from cyclomethicones and dimethicones and mixtures thereof. These materials are known in the art and are often commercially available. Thus one of the preferred oil phases can be considered, and is thus defined as a "silicone" phase and thus the foundation is defined as a "water-in-silicone" emulsion.

In the "water-in-silicone" emulsion embodiment, a silicone is used in the oil phase and the silicone can comprise one or more volatile silicones, non-volatile silicones, and mixtures of volatile silicones and non-volatile silicones. The silicone is present in an amount of from about 1% to about 50% by weight of the foundation. Suitable volatile silicones include cyclic and linear volatile polyorganosiloxanes (as used herein, "volatile" refers to those materials which have a measurable vapor pressure at ambient conditions). A description of various volatile silicones is found in Todd, et al.. "Volatile Silicone Fluids for Cosmetics", 91 *Cosmetics and Toiletries* 27-32 (1976). Preferred volatile silicones can include cyclic and linear polydimethylsiloxanes.

Preferred volatile linear silicones generally have viscosities of less than about 5 centistokes at 25°C, while preferred volatile cyclic silicones typically have viscosities of less than about 10 centistokes. Some examples of volatile silicones useful in the present invention include: Dow Coming's 344, 345, 244, 245, and 200 silicones (manufactured by the Dow Corning Corporation): Silicone 7207 and Silicone 7158 (manufactured by the Union Carbide Corporation). SF1202 (manufactured by General Electric), Siloxane 5223 (available from Wacker Silicones) and the like: Of course, others are available and known in the art.

Suitable non-volatile silicones preferably have an average viscosity of from about 10 to about 2,000,000 centistokes at 25°C, more preferably from about 10 to about 50,000 centistokes, even more preferably from about 50 to about 5000 centistokes. Of course, higher viscosity non-volatile silicone conditioning agents can also be used. Suitable non-volatile silicone fluids include, for example, polyalkyl siloxanes, polyaryl siloxanes, polyalkylaryl siloxanes, polysiloxanes with amino functional substitutions, polyether siloxane copolymers, and mixtures thereof. Preferred non-volatile siloxane fluids that may be used include, for example, polydimethylsiloxanes, polymethylphenylsiloxanes polyalkyl siloxanes and polyether siloxane copolymers and the like. These siloxanes are available, for example, from Dow Corning, as the Dow Coming 200 series, Dow Coming DC-1248, Dow Coming DC-593, 556 Cosmetic Grade Fluid, Dow Coming 2502 or from the General Electric Company, as SF 1075 methyl phenyl fluid, SF1202 and the like. References disclosing suitable silicone fluids include US patent 2,826,551 (publication date: March 11,1958/inventor: Geen); US patent 3,964,500 (publication date: June 22,1976/inventor: Drakoff); US patent 4,364,837 (publication date:December 21,1982/inventor: Pader), incorporated herein by reference. In addition, *Silicone Compounds* distributed by Petrarch Systems Inc., 1984 provides an extensive (although not exclusive) listing of suitable silicone fluids. Other suitable and preferred silicones are disclosed in U.S. Patent 5,143,722 (publication date: September 1, 1992/inventors: Hollenberg, Lombardi and Tietjen), incorporated herein by reference.

The oil phase can additionally comprise oil soluble components, such as emulsifiers, colorants or pigments, emollients, fragrances, waxes, stabilizers, dispersants and the like.

### B. Water Soluble or Dispersible Polymer

The water soluble or dispersible polymer used in the foundation composition of the invention is compatible with the aqueous phase of the emulsion, and is incorporated in the internal phase of the water-in-oil emulsion, rather than in simple solution or in an oil-in-water emulsion. The polymer can be water dispersible, or water soluble, but is not a cross linked or a water swellable polymer. The polymer may form a thin elastomeric film that physically adheres or interacts with the skin. The polymer film, when formed, must also be water removable, i.e., easily removable with water and soap. Preferably, the polymers which do not bestow a tacky feel are employed.

The water soluble or dispersible polymer is formulated in the aqueous phase of the emulsion. The polymer is selected to provide a finished foundation preferably with a glass transition temperature (Tg) of about room temperature to about body temperature. "Glass transition temperature" or "Tg" refers to the temperature where the polymer softens or transitions from brittle to plastic, in the absence of plasticizers. This provides for a flexible polymer during application and wear. When the Tg is too high, the foundation may be hard to apply, and may flake. If it is too low, the foundation will be less adhesive (and perhaps more cohesive) and will tend to "ball up" on application.

Of course, the Tg of the polymer itself can vary. For example, it is expected that polymers with Tg of up to about 60°C or higher are useful, provided the finished formulation has the proper Tg. For example polyvinylpyrrolidone is thought to have a Tg greater than 90°C, but is useful in the invention. Typical polymers used in the invention are thermoplastic, rather than thermosetting.

Additionally, the polymer should be selected to provide an aqueous phase that is fluid enough to be handled and reasonably incorporated into the final emulsion composition as the dispersed or internal phase. Gelled and extremely viscous solutions can be used, but may impact ease of incorporation or final viscosity. Thus it is preferred to select polymers which can be added at levels to derive film forming and extended benefits, while maintaining workability of the final aqueous phase.

Examples of preferred polymers that have acceptable Tg, skin adhering properties and viscosity include, sulfopolyester resins, such as AQ sulfopolyester resins, such as AQ29D, AQ35S, AQ38D, AQ38S, AQ48S, and AQ55S (available from Eastman Chemicals), Vinex resins (polyvinylacetate/polyvinyl alcohol resins), such as Vinex 2034, Vinex 2144, and Vinex 2019 (available from Air Products), Dermacryl acrylic resins (water dispersible acrylic resins/ available from National Starch), polyvinylpyrrolidones (PVP), including Luviskol K17, K30 and K90 (available from BASF), water soluble copolymers of PVP, including PVP/polyvinyl alcohol copolymer, PVP/VA S-630 and W-735 and PVP/dimethylaminoethylmethacrylate Copolymers such as Copolymer 845 and Copolymer 937 available from ISP, and the like. A preferred polymer is PVP.

If the polymers are present in levels of more than 10%, it results in an undesirable level of tackiness. If the polymers are present in levels of less than 0.1%, the foundation may not be sufficiently stable.

Typically, the polymer is present in levels of from about 0.1% to about 10% by weight of the foundation. More preferably, the polymer level is from about 0.5% to about 8% by weight of the foundation.

In order to form a thin elastomeric film, the polymer level is at least about 0.5% by weight of the foundation.

Using the parameters defined above, and depending upon the choice of polymer, the preferred level of the polymer may vary. For example, when PVP is used as the film forming polymer, a still more preferred level is from about 0.5% to about 5% by weight. As another example, when the sulfopolyester AQ38S is used, the still more preferred level is from about 2% to about 8% by weight.

As used herein, the term "sulfopolyester resins," "sulfopolyester resin" or "AQ resin" refers to any of the AQ sulfopolyester resins, such as AQ29D, AQ35S, AQ38D, AQ38S, AQ48S and AQ55S, available from Eastman Chemicals (Rochester, New York, U.S.A.), as described above.

As used herein, the term polyvinylacetate/polyvinyl alcohol resins refers to such polymers as they are known in the art. Preferred examples of these are referred to as "Vinex" or "Vinex resins", available from Air Products,- such as Vinex 2034, Vinex 2144, and Vinex 2019 described above.

Water dispersible acrylic resins are known in the art. "Dermacryl" is a preferred family of such water dispersible acrylic resins, available from National Starch, as Dermacryl LT and the like.

Polyvinylpyrrolidones are known in the art. Their description, characterization and commercial designations are disclosed by E.S. Barabas in the Encyclopedia of Polymer Science and Engineering, 2 Ed. Vol. 17 pp. 198-257.

### C. Plasticizing Solvent

The term "plasticizing solvent," as used herein includes slow evaporating, water miscible or dispersible cosolvents that are 1) generally recognized as safe (GRAS: Generally recognized as safe), many of these are listed in C.T.F.A. Cosmetic Ingredient Handbook, 3rd Ed., Cosmetic and Fragrance Assn., Inc., Washington D.C. (1982) pp. 575-580, incorporated herein by reference, or 2) include slow evaporating glycols and glycol ethers including for example, propylene glycol, butylene glycol, hexylene glycol, glycerin, dipropylene glycol, dipropylene glycol methyl ether (commonly known as DPM), propylene glycol phenyl ether, and polyethylene glycols (PEGs) such as PEG 4 and PEG 8. Other classes of solvents include, propylene carbonate, and dimethyl isosorbide and mixtures thereof. More preferred solvents include propylene glycol, butylene glycol, dipropylene glycol, glycerin and mixtures thereof.

If the solvents are present in levels of more than 30%, it results in an undesirable level of tackiness. If the solvents are present in levels of less than 0.5%, sufficient solubility, stability, low volatility effect, and suitable feel and appearance during application cannot be expected.

These solvents are preferably present in the foundations of the present invention in an amount of from about 0.5% to about 30%, more preferably from about 5% to about 20%. The solvents preferably appear in a ratio of solvent to polymer of from about 10:1 to 1:1, more preferably of from about 8:1 to 2:1. Exact levels and ratios can be adjusted depending upon desired solvation, evaporation rate and the like.

The plasticizing solvent is chosen to provide for water co-solvency, suitable solubility regarding the polymer, low volatility, stability and of course safety (i.e., lack of toxicity). Thus the foundation employs safe solvents that provide little or no sensation of tackiness, or cooling (usually due to evaporation) on the applied area. For example, any of the glycols are contemplated to be useful, including polyethylene glycols. These solvents also can be dipolar aprotic solvents that minimize hydrogen bonding and concomitant gelling and the like. For example, DMSO (dimethyl sulfoxide) or DMF (N,N-dimethylformamide) would be acceptable solvents, but for the safety concerns with the solvents.

Typically the preferred polymer and plasticizing solvent are chosen such that the polymer and plasticizing solvent are in the aqueous phase of the emulsion. This diminishes any tacky sensation of polymer contacting the user's hands and fingers during initial application of the cosmetic. Typically the pigment is in the oil phase for evenness. The solvent is chosen for its slow evaporation rate and its presence in the aqueous phase, and solvation properties. Typically the solvent also extends the workability of the foundation and delays any perceived onset of tackiness for as long as possible, preferably up to two minutes.

### D.The Ultra Fine Titanium Dioxide-Water Dispersion

The ultra fine titanium dioxide-water dispersion is useful for obtaining a cosmetic having adequate coverage effect and high SPF value with a minimum amount of the ultra fine titanium oxide.

An ultra fine titanium dioxide-water dispersion useful in the present invention is disclosed in Japanese patent laid-open No. 7-247119 (publication date: September 26, 1995/ inventors:.Okuda, Futamata and Iida)

The ultra fine titanium dioxide-water dispersion comprises water, ultra fine titanium dioxide and a nonionic surfactant as a dispersant.

If the foundation comprises more than 30% by weight of the foundation, of the ultra fine titanium dioxide-water dispersion, the foundation cannot maintain adequate coverage and sufficient spreadability. If the foundation comprises less than 1% by weight of the foundation of the ultra fine titanium dioxide-water dispersion, the foundation will not have sufficient SPF value. The foundation preferably comprises the ultra fine titanium dioxide-water dispersion in an amount of from about 1% to about 30%, more preferably from about 5% to about 10%, by weight of the foundation.

The ultra fine titanium dioxide preferably has an average particle size of from about 10 nm to about 100nm.

Preferably, the particle surface of the ultra fine titanium dioxide is hydrophobically treated to make the particles hydrophobic by an agent providing the hydrophobic property which is selected from the group consisting of siloxanes, silane-coupling agents, titanate-coupling agents, aluminum-coupling agents, fluorine-coupling agents, higher fatty acids, higher alcohols, amines having at least one alkyl group having at least six carbon atoms, and mixture thereof. The agent providing the hydrophobic property is present in levels of from about 0.5% to about 8%, preferably from about 2% to about 5%, by weight of the ultra fine titanium dioxide.

The ultra fine titanium dioxide is preferably covered by an oxide or hydroxide of metal which is selected from the group consisting of aluminum, silica, zirconium, titanium, zinc and stannum before a hydrophobic property is given to the particle surface of the ultra fine titanium dioxide.

If the ultra fine titanium dioxide-water dispersion comprises more than about 18% by weight of the foundation of the ultra fine titanium dioxide, the ultra fine titanium dioxide agglomerates. If the water dispersion comprises less than about 0.2% by weight of the foundation of the ultra fine titanium dioxide, sufficient SPF value of a foundation will not be obtained.

The ultra fine titanium dioxide-water dispersion preferably comprises from about 0.2% to about 18% by weight of the foundation (from about 20% to about 60% by weight of the water dispersion), of an ultra fine titanium dioxide.

Useful nonionic surfactants include polyglycerin fatty acid esters, propylene glycol fatty acid esters, glycerin fatty acid esters, sorbitan fatty acid esters, sugar fatty acid esters, polyoxyethylene sorbitan fatty acid esters, polyoxyethylene sorbit fatty acid esters, polyethylene glycol fatty acid esters, polyoxyethylene glycerin fatty acid esters, polyoxyethylene castor oils, polyoxyethylene hardened castor oils, polyoxyethylene alkyl ethers, polyoxyethylene phytosterols, polyoxyethylene polyoxypropylene alkyl ethers, polyoxyethylene alkyl phenyl ethers, polyoxyethylene lanolins, polyoxyethylene lanolin alcohols, polyoxyethylene beeswax derivatives, polyoxyethylene fatty acid amides, dimethicone copolyols(silicone glycol copolymers), and polyoxyethylene fatty acid esters. Preferred nonionic surfactants are polyoxyethylene sorbitan fatty acid esters, polyoxyethylene alkyl ethers, dimethicone copolyols, sorbitan fatty acid esters, and sugar fatty acid esters. More preferred nonionic surfactants are dimethicone copolyols and polyoxyethylene sorbitan fatty acid esters.

The level of nonionic surfactant depends on the particle size of titanium dioxide, kinds of nonionic surfactant, and so on. However, if the ultra fine titanium dioxide-water dispersion comprises more than about 7.2% by weight of the foundation of the nonionic surfactant, it will cause sticky feel of the foundation. If the water dispersion comprises less than 0.002% by weight of the foundation of the nonionic surfactant, the ultra fine titanium dioxide agglomerates.

The level of nonionic surfactant is preferably from about 0.002% to about 7.2% by weight of the foundation (from about 1% to about 40% by weight of the ultra fine titanium dioxide).

### E.Other Ingredients

### a)Pigments, Colorants and Fillers

There are no specific limitations as to the pigment, colorant or filler powders used in the foundation composition. Each may be a body pigment, inorganic white pigment, inorganic colored pigment, pearling agent, and the like. Specific examples are talc, mica, magnesium carbonate, calcium carbonate, magnesium silicate, aluminum magnesium silicate, silica, titanium dioxide, zinc oxide, red iron oxide, yellow iron oxide, black iron oxide, ultramarine, nylon powder, polyethylene powder, methacrylate powder, polystyrene powder, silk powder, crystalline cellulose, starch, titanated mica, iron oxide titanated mica, bismuth oxychloride, and the like. These pigments and powders can be used independently or in combination.

The pigments are preferably used as opacifiers and colorants. They are present in a concentration sufficient to provide a pleasing color to the composition in the container in which the foundation is sold and to confer the desired coverage and color to skin when applied. These pigments can be used as treated particles, or as the raw pigments themselves. Typical pigment levels are selected for the particular purpose of the foundation. For example, a foundation for fair skinned individuals would typically use lighter pigments and those pigments in a lower amount, while a foundation for darker skinned individuals might require darker pigmentation and/or more pigmentation. Determination of these levels and pigment types is within the skill of the artisan. Pigments that are generally recognized as safe, and are listed in C.T.F.A. Cosmetic Ingredient Handbook, 3rd Ed., Cosmetic and Fragrance Assn., Inc., Washington D.C. (1982), incorporated herein by reference, are used.

It is preferred that the pigments are surface treated to provide added stability of color and ease of formulation. Hydrophobically treated pigments are more preferred, because they may be more easily dispersed in the oil phase. In addition, it may be useful to treat the pigments with a material that is compatible with that silicone phase. Particularly useful hydrophobic pigment treatments for use in water-in-silicone emulsions include polysiloxane treatments such as those disclosed in US Patent 5,143,722(publication date: September 1, 1992/inventors: Hollenberg, Lombardi and Tietjen) incorporated herein by reference.

Filler powders can be used to modify the density, feel or thickness of the composition or as a matte finishing agent to hide skin defects and reduce shine. Such cosmetically acceptable agents include those disclosed in the C.T.F.A. Cosmetic Ingredient Handbook, 3rd Ed., Cosmetic and Fragrance Assn., Inc., Washington D.C. (1982), incorporated herein by reference. For example, spherical silica, hydrated silica, silicone-treated silica beads, mica, talc, polyethylene, bentonite, hectorite, kaolin, chalk, diatomaceous earth, attapulgite and the like may be utilized. Of the components useful as a matte finishing agents, low luster pigment, talc, polyethylene, hydrated silica, kaolin, titanium dioxide, titanated mica (mica coated with titanium dioxide) and mixtures thereof are preferred.

### b) Emulsifiers

The hydrophilic-lipophilic balance value of the emulsifier (herein referred to as HLB) is chosen so as to provide a water-in-oil emulsion. This factor is referenced in Wilkinson and Moore, Harry's Cosmeticology, 7th Ed. 1982, p. 738. and Schick and Fowkes, Surfactant Science Series, Vol. 2, Solvent Properties of Surfactant Solutions, p 607, incorporated herein by reference. HLB values of surfactant emulsifiers for making water-in-oil emulsions are from about 3-6. These emulsifiers include those disclosed in the C.T.F.A. Cosmetic Ingredient Handbook, 3rd Ed., Cosmetic and Fragrance Assn., Inc., Washington D.C. (1982) pp. 587-592; and Remington's Pharmaceutical Sciences, 15th Ed. 1975, pp. 335-337; both incorporated herein by reference. These emulsifiers are known in the art, and mixtures of these can be used, including those in McCutcheon's Volume 1, Emulsifiers & Detergents, 1994, North American Edition, pp. 236-239; incorporated herein by reference.

Particularly useful emulsifiers for water-in-silicone emulsions include polydiorganosiloxane-polyoxyalkylene copolymers. Such polymers are described in US patent 4,268,499(publication date:May 19,1981/inventor: Keil), incorporated herein by reference. Suitable copolymers are known and many are available commercially. A preferred emulsifier herein is known by its CTFA designation as dimethicone copolyol. Preferred emulsifiers are further disclosed by U. S. Patent 5,143,722(publication date: September 1, 1992/inventors: Hollenberg, Lombardi and Tietjen), incorporated herein by reference. The foundation may comprise from about 0.5% to about 10%, preferably from about 1% to about 5%, more preferably from about 1.5% to about 3% of one or more emulsifiers.

### c) Waxes

Optionally, the foundation may contain one or more waxes to affect viscosity, feel or stability. Waxes are lower-melting organic mixtures or compounds of high molecular weight, solid at room temperature and generally similar in composition to fats and oils except that they contain no glycerides. They can be hydrocarbons, esters of fatty acids, or alcohols. Waxes useful in the present invention are selected from the group consisting of animal waxes, vegetable waxes, mineral waxes, natural waxes, synthetic waxes, petroleum waxes, ethylenic polymers, hydrocarbons, silicone waxes, and mixtures thereof.

### d) Moisturizers

Optionally, one or more moisturizing agents can be used in the foundation composition. Among the moisturizing agents useful in the foundation composition are such well known cosmetically effective moisturizing agents as glycerin, hydrogen starch hydrolysate, sorbitol, hydrolyzed silk and the like.

These agents are defined in the C.T.F.A. Cosmetic Ingredient Handbook, 3rd Ed., Cosmetic and Fragrance Assn., Inc., Washington D.C. (1982)., incorporated herein by reference.

### e) Fragrances

In addition, a fragrance, is optionally present in the above foundation composition in a concentration to provide a light, pleasant scent during application and/or to mask any odors of the composition.

### f) Preservatives

Typically preservatives, such as those listed in C.T.F.A. Cosmetic Ingredient Handbook, 3rd Ed., Cosmetic and Fragrance Assn., Inc., Washington D.C. (1982) pp. 575-580, incorporated herein by reference, are used in a foundation. The levels of these preservatives are readily determined by those skilled in the art. For example, favored levels are often less than 5 % and typically less than 1%.

### g) Miscellaneous Ingredients

Other materials can be incorporated into the formulation without departing from the scope or spirit of the invention. For example, materials may be added to provide added stability at storage temperature, such as thickeners and the like; better feel upon application, such as waxes; increased viscosity; coloration when wet; and the like.

Water dispersible and oil dispersible clays may be useful to thicken the water or the oil phase of the invention. The water dispersible clays comprise bentonite and hectorite, such as Bentone EW, LT from Rheox; magnesium aluminum silicate, such as Veegum from Vanderbilt Co.; attapulgite such as Attasorb or Pharamasorb from Engelhard, Inc.; laponite and montmorrilonite, such as Gelwhite from ECC America; and mixtures thereof. The oil dispersible clays comprise quaternium-18 bentonite, such as Bentone 34 and 38 from Rheox; the Claytone Series from ECC America; quaternium-18 hectorite, such as Bentone gels from Rheox; and mixtures thereof. Other particulate or organic thickeners may also be useful provided they do not compromise the function or aesthetics of the foundation.

Electrolytes such as sodium chloride, may be useful to stabilize the emulsion.

Dispersants, such as ester oil and fatty acid ester, may be useful to disperse pigments

Another optional component comprises one or more ultraviolet absorbing agents. Ultraviolet absorbing agents, often described as sunscreening agents, can be present in a concentration in the range of between about 1% and about 12% by weight, based on the total weight of composition.

### F. Process For Preparing a Water-in-oil Emulsion Foundation

The present invention also relates to a process for preparing a stable water-in-oil emulsion foundation comprising the steps of (a) mixing oil soluble components to obtain an oil mixture, (b) mixing a water soluble or dispersible polymer, a plasticizing solvent and water soluble additives to obtain an aqueous mixture, (c) mixing the aqueous mixture and the oil mixture together to obtain an emulsion, and (d) mixing the emulsion and an ultra fine titanium dioxide-water dispersion together, wherein the water dispersion comprises an ultra fine titanium dioxide and a nonionic surfactant.

The oil-soluble components include silicones such as volatile silicones and non-volatile silicones, emulsifiers, colorants or pigments, fragrances, waxes, dispersants, oil soluble stabilizers, oil-soluble preservatives and the like. The oil-soluble components preferably comprise a silicone, an emulsifier, a colorant or pigment and a dispersants. If the foundation comprises more than about 80% by weight of the foundation of the oil-soluble components, it will cause sticky feel of the foundation. If the foundation comprises less than about 20% by weight of the foundation of the oil-soluble components, the foundation made by the process cannot be emulsified. The level of the oil-soluble components is preferably from about 20% to about 80%, more preferably from about 40% to about 70%, by weight of the foundation.

The water soluble additives include moisturizers, electrolytes, water, water-soluble preservatives and the like. The water-soluble additives preferably comprise a moisturizer, an electrolyte, and water. If the foundation comprises more than about 75% by weight of the foundation of the water-soluble additives, the foundation made by the process cannot be emulsified. If the foundation comprises less than about 10% by weight of the foundation of the water-soluble additives, it will cause sticky feel of the foundation. The level of the water-soluble additives is from about 10% to about 75%, preferably from about 15% to about 50%, by weight of the foundation.

The water soluble or dispersible polymer, the plasticizing solvent, and the ultra fine titanium dioxide-water dispersion comprising an ultra fine titanium dioxide and a nonionic surfactant are as previously described above.

The mixing in the steps of (a), (b), (c) and (d) can be conducted by using a homomixer and anchor mixer for at least 30 minutes.

In an embodiment where the foundation comprises a silicone, if the process of the present invention is conducted at a temperature of more than 30°C, the emulsion may become unstable. If the process of the present invention is conducted at a temperature of less than 23° C, the solubility of the oil may become worse. Preferably, process of the present invention is conducted at a temperature of from about 23°C to about 30°C, more preferably from about 25 °C to about 28°C if the foundation comprises a silicone; otherwise at a temperature of from about 23°C to about 98°C, more preferably from about 25°C to about 95°C.

### G. Examples

The following examples further describe and demonstrate embodiments within the scope of the present invention. The examples are given solely for the purpose of illustration. All percentages are based on weight.

Example compositions numbers 1-6 are shown in Table 1 as Ex 1-6.

In these examples, the small variation in the oil phase is not thought to alter the properties of the formulas.

**Table 1**

| Ingredient | Ex 1 | Ex 2 | Ex 3 | Ex 4 | Ex 5 | Ex 6 |
|---|---|---|---|---|---|---|
| Oil Phase | | | | | | |
| Emulsifiers | 2.25 | 2.25 | 2.25 | 2.25 | 2.25 | 2.50 |
| non-volatile liquids | 5.00 | 5.00 | 5.00 | 5.00 | 5.00 | 5.00 |
| Volatile Silicones | 30.50 | 30.50 | 30.50 | 30.5 | 30.5 | 50.00 |
| Pigments and Fillers | 15.50 | 15.50 | 15.50 | 15.50 | 15.50 | 15.50 |
| Rheological Additives/ Fragrances/Preservatives | 1.00 | 1.00 | 1.00 | 1.00 | 1.00 | 1.00 |

| Aqueous Phase | | | | | | |
|---|---|---|---|---|---|---|
| Deionized Water | 29.33 | 26.83 | 24.33 | 27.33 | 14.83 | 7.08 |
| Film forming polymer resin (1) | 1.00 | 1.00 | 1.00 | 0.50 | 1.00 | 1.00 |
| Methyl Paraben | 0.12 | 0.12 | 0.12 | 0.12 | 0.12 | 0.12 |
| Propylene Glycol | 8.00 | 8.00 | 8.00 | 8.00 | 20.00 | 8.00 |
| Sodium Dehydroacetate | 0.30 | 0.30 | 0.30 | 0.30 | 0.30 | 0.30 |
| Sodium Chloride | 2.00 | 2.00 | 2.00 | 2.00 | 2.00 | 2.00 |
| Ultra fine Titanium Dioxide water-dispersion(2) | 5.00 | 7.50 | 10.00 | 7.50 | 7.50 | 7.50 |
| Total | 100.00 | 100.00 | 100.00 | 100.00 | 100.00 | 100.00 |

| Ref | Ingredient Name | Supplier |
|---|---|---|
| 1 | PVP K-17 | BASF |
| 2 | Titanium Dioxide TTW-2 | Ishihara Sangyo |

The example compositions numbers 1-6 (Ex 1-6) were prepared by the following method: Disperse pigments in oil phase liquids using high speed disperser, high shear mill or other methods known in the art to ensure uniform color and efficient use of pigment. Add the remainder of the ingredients in the oil phase and sheer until well mixed, heating if necessary to ensure any solid waxes are melted. Combine all aqueous phase ingredients, (except polymer), with mixing. Add the polymers to the aqueous phase using methods described in their supplier's literature. Cool both phases to room temperature and slowly add the aqueous phase to the oil phase, mixing with stirrer, homogenizer or other methods known in the art to form an emulsion. After the emulsification is completed, add the ultra fine titanium dioxide water-dispersion to the emulsion, mixing with stirrer, homogenizer or other methods. Final properties of the emulsion such as viscosity may be adjusted by amount and type of mixing as would be evident to one skilled in the art.

The compositions of the present invention have improved stability compared versus compositions which do not comprise a water soluble or dispersible polymer. An improved SPF value is obtained by the compositions of the present invention even though the compositions do not comprise any organic UV absober. The compositions do not spoil spreadability and suitable feel and appearance during application.

It is understood that the examples and embodiments described herein are for illustrative purposes only and that various modifications or changes in light thereof will be suggested to one skilled in the art and are to be included in the spirit and purview of this application and scope of the appended claims.

## Claims

1. A water-in-oil emulsion foundation wherein the aqueous internal phase comprises:
a) from 0.1% to 10% by weight of the foundation, of a water soluble or dispersible polymer;
b) from 0.5% to 30 % by weight of the foundation, of a plasticizing solvent; and
c) from 1% to 30% by weight of the foundation of an ultra fine titanium dioxide-water dispersion, wherein the ultra fine titanium dioxide-water dispersion comprises from 0.2% to 18% by weight of the foundation of an ultra fine titanium dioxide, and from 0.002% to 7.2% by weight of the foundation of a nonionic surfactant.

2. The foundation of claim 1 wherein the nonionic surfactant is selected from the group consisting of polyoxyethylene sorbitan fatty acid esters, polyoxyethylene alkyl ethers, dimethicone copolyols, sorbitan fatty acid esters, sugar fatty acid esters and mixtures thereof.

3. The foundation of claim 1 wherein the nonionic surfactant is selected from the group consisting of dimethicone copolyols, polyoxyethylene sorbitan fatty acid esters and mixtures thereof.

4. The foundation of claim 1 wherein the plasticizing solvent is selected from the group consisting of propylene glycol, butylene glycol, dipropylene glycol, glycerin and mixtures thereof.

5. The foundation of claim 1 wherein the polymer is selected from the group consisting of sulfopolyester resins, water dispersible acrylic resins, polyvinylacetate/polyvinyl alcohol resins, polyvinylpyrrolidone, polyvinylpyrrolidone/polyvinyl alcohol copolymers, and mixtures thereof.

6. The foundation of claim 1 wherein the polymer is present in an amount of from 0.5% to 8% by weight of the foundation.

7. The foundation of claim 6 wherein
(i) the plasticizing solvent is present in an amount of from 5% to 20% by weight of the foundation and the solvent is propylene glycol, and
(ii) the polymer is present in an amount of from 0.5% to 8% by weight of the foundation and the polymer is polyvinylpyrrolidone.

8. The foundation of claim 6 wherein
(i) the ultra fine titanium dioxide has an average particle size of from about 10 nm to about 100nm, and
(ii) the particle surface of the ultra fine titanium dioxide has been hydrophobically treated to make the particles hydrophobic by an agent selected from the group consisting of siloxanes, silane-coupling agents, titanate-coupling agents, aluminum-coupling agents, fluorine-coupling agents, higher fatty acids, higher alcohols, amines having at least one alkyl group having at least six carbon atoms, and mixtures thereof.

9. The foundation of claim 8 wherein the ultra fine titanium dioxide is covered by an oxide or hydroxide of metal which is selected from the group consisting of aluminium, silica, zirconium, titanium, zinc and stannum.

10. A process for preparing a stable water-in-oil emulsion foundation comprising the steps of
(a) mixing oil soluble components to obtain an oil mixture,
(b) mixing a water soluble or dispersible polymer, a plasticizing solvent and water soluble additives to obtain an aqueous mixture,
(c) mixing the aqueous mixture and the oil mixture together to obtain an emulsion, and
(d) mixing the emulsion and an ultra fine titanium dioxide-water dispersion together, wherein the ultra fine titanium dioxide-water dispersion comprises an ultra fine titanium dioxide and a nonionic surfactant.

11. The process of claim 10 wherein the oil-soluble components comprise a silicone, an emulsifier, a colorant or pigment and a dispersing agent.

12. The process of claim 10 wherein the water soluble additives comprise a moisturizer, an electrolyte, and water.

13. The process of claim 10 wherein a silicone is present in the oil-soluble components and all steps are conducted at a temperature of from 23°C to 30°C.

14. The process of claim 10 wherein the nonionic surfactant is selected from the group consisting of dimethicone copolyols and polyoxyethylene sorbitan fatty acid esters.

15. The process of claim 10 comprising the steps of
(a) mixing from 20% to 80% by weight of the foundation, of oil-soluble components to obtain an oil mixture,
(b) mixing from 0.1% to 10% by weight of the foundation, of a water-soluble or water-dispersible polymer; from 0.5% to 30% by weight of the foundation, of a plasticizing solvent; and from 10% to 75% by weight of the foundation, of water soluble additives; to obtain an aqueous mixture,
(c) mixing the aqueous mixture and the oil mixture together to obtain an emulsion, and
(d) mixing the emulsion and from 1% to 30% by weight of the foundation of an ultra fine titanium dioxide-water dispersion together, wherein the ultra fine titanium dioxide-water dispersion comprises from 0.2% to 18% by weight of the foundation of an ultra fine titanium dioxide, and from 0.002% to 7.2% by weight of the foundation of a nonionic surfactant.

16. The process of claim 15 wherein the water-soluble additives comprise a moisturizer, an electrolyte and water.

17. The process of claim 15 wherein the polymer is selected from the group consisting of sulfopolyester resins, water dispersible acrylic resins, polyvinylacetate/polyvinyl alcohol resins, polyvinylpyrrolidone, polyvinylpyrrolidone/polyvinyl alcohol copolymers, and mixtures thereof.

18. The process of claim 15 wherein the polymer is present in an amount of from 0.5% to 8% by weight of the foundation and the polymer is polyvinylpyrrolidone.

19. The process of claim 15 wherein (i) the ultra fine titanium dioxide has an average particle size of from about 10 nm to about 100nm, and (ii) the particle surface of the ultra fine titanium dioxide has been treated hydrophobically to make the particles hydrophobic by an agent selected from the group consisting of siloxanes, silane-coupling agents, titanate-coupling agents, aluminum-coupling agents, fluorine-coupling agents, higher fatty acids, higher alcohols, amines having at least one alkyl group having at least six carbon atoms, and mixture thereof.

20. The process of claim 19 wherein the ultra fine titanium dioxide is covered by an oxide or hydroxide of metal which is selected from the group consisting of aluminium, silica, zirconium, titanium, zinc and stannum.

## Revendications

1. Fond de teint en émulsion eau dans huile, dans lequel la phase aqueuse interne contient :
a) 0,1 % à 10 % en poids du fond de teint d'un polymère soluble ou dispersible dans l'eau ;
b) 0,5% à 30 % en poids du fond de teint d'un solvant plastifiant, et
c) 1% à 30 % en poids du fond de teint d'une dispersion dioxyde de titane ultrafin et eau, la dispersion dioxyde de titane ultrafin et eau contenant 0,2 % à 18 % en poids du fond de teint d'un dioxyde de titane ultrafin et 0,002 % à 7,2 % en poids du fond de teint d'un tensioactif non ionique.

2. Fond de teint selon la revendication 1, dans lequel le tensioactif non ionique est choisi dans le groupe constitué des esters d'acides gras de polyoxyéthylène sorbitane, des alkyléthers de polyoxyéthylène, des copolyols de diméthicone, des esters d'acides gras de sorbitane, des esters d'acides gras de sucre et de mélanges de ceux-ci.

3. Fond de teint selon la revendication 1, dans lequel le tensioactif non ionique est choisi dans le groupe constitué des copolyols de diméthicone, des esters d'acides gras de polyoxyéthylène sorbitane et de mélanges de ceux-ci.

4. Fond de teint selon la revendication 1, dans lequel le solvant plastifiant est choisi dans le groupe constitué du propylène glycol, du butylène glycol, du dipropylène glycol, de la glycérine et de mélanges de ceux-ci.

5. Fond de teint selon la revendication 1, dans lequel le polymère est choisi dans le groupe constitué des résines sulfopolyester, des résines acryliques dispersibles dans l'eau, des résines polyvinylacétate/alcool de polyvinyle, de la polyvinylpyrrolidone, des copolymères polyvinylpyrrolidone/alcool de polyvinyle et de mélanges de ceux-ci.

6. Fond de teint selon la revendication 1, dans lequel le polymère est présent dans une quantité comprise entre 0,5 % et 8 % en poids du fond de teint.

7. Fond de teint selon la revendication 6, dans lequel
(i) le solvant plastifiant est présent dans une quantité comprise entre 5 % et 20 % en poids du fond de teint, et dans lequel le solvant est le propylène glycol, et
(ii) le polymère est présent dans une quantité comprise entre 0,5 % et 8 % en poids du fond de teint et dans lequel le polymère est la polyvinylpyrrolidone.

8. Fond de teint selon la revendication 6, dans lequel
(i) le dioxyde de titane ultrafin présente une taille moyenne des particules comprise entre environ 10 nm et environ 100 nm, et
(ii) la surface des particules du dioxyde de titane ultrafin a été traitée de façon hydrophobe pour rendre hydrophobes les particules par un agent choisi dans le groupe constitué de siloxanes, d'agents de couplage du silane, d'agents de couplage du titanate, d'agents de couplage de l'aluminium, d'agents de couplage du fluor, d'acides gras supérieurs, d'alcools supérieurs, d'amines ayant au moins un groupe alkyle comprenant au moins six atomes de carbone, et de mélanges de ceux-ci.

9. Fond de teint selon la revendication 8, dans lequel le dioxyde de titane ultrafin est recouvert d'un oxyde ou d'un hydroxyde de métal qui est choisi dans le groupe constitué de l'aluminium, de la silice, du zirconium, du titane, du zinc et de l'étain.

10. Procédé de préparation d'un fond de teint en émulsion eau dans huile stable comprenant les étapes consistant à
a) mélanger les composants liposolubles de façon à obtenir un mélange huileux,
b) mélanger un polymère soluble ou dispersible dans l'eau, un solvant plastifiant et des additifs hydrosolubles pour obtenir un mélange aqueux,
c) mélanger le mélange aqueux et le mélange huileux pour obtenir une émulsion, et
d) mélanger l'émulsion et une dispersion dioxyde de titane ultrafin et eau, la dispersion dioxyde de titane ultrafin et eau contenant un dioxyde de titane ultrafin et un tensioactif non ionique.

11. Procédé selon la revendication 10, dans lequel les composants liposolubles comprennent une silicone, un émulsifiant, un colorant ou un pigment et un agent dispersant.

12. Procédé selon la revendication 10, dans lequel les additifs hydrosolubles comprennent un agent hydratant, un électrolyte et de l'eau.

13. Procédé selon la revendication 10, dans lequel une silicone est présente dans les composants liposolubles et dans lequel toutes les étapes sont réalisées à une température comprise entre 23 °C et 30 °C.

14. Procédé selon la revendication 10, dans lequel le tensioactif non ionique est choisi dans le groupe constitué de copolyols de diméthicone et d'esters d'acides gras de polyoxyéthylène sorbitane.

15. Procédé selon la revendication 10, comprenant les étapes consistant à
a) mélanger 20 % à 80 % en poids du fond de teint des composants liposolubles pour obtenir un mélange huileux,
b) mélanger 0,1% à 10% en poids du fond de teint d'un polymère soluble ou dispersible dans l'eau ; 0,5 % à 30 % en poids du fond de teint d'un solvant plastifiant; et 10 % à 75 % en poids du fond de teint d'additifs hydrosolubles, pour obtenir un mélange aqueux,
c) mélanger le mélange aqueux et le mélange huileux pour obtenir une émulsion, et
d) mélanger l'émulsion et 1 % à 30 % en poids du fond de teint d'une dispersion dioxyde de titane ultrafin et eau, la dispersion dioxyde de titane ultrafin et eau contenant 0,2 % à 18 % en poids du fond de teint d'un dioxyde de titane ultrafin et 0,002 % à 7,2 % en poids du fond de teint d'un tensioactif non ionique.

16. Procédé selon la revendication 15, dans lequel les additifs hydrosolubles comprennent un agent hydratant, un électrolyte et de l'eau.

17. Procédé selon la revendication 15, dans lequel le polymère est choisi dans le groupe constitué des résines sulfopolyester, des résines acryliques dispersibles dans l'eau, des résines polyvinylacétate/alcool de polyvinyle, de la polyvinylpyrrolidone, des copolymères polyvinylpyrrolidone/alcool de polyvinyle et de mélanges de ceux-ci.

18. Procédé selon la revendication 15, dans lequel le polymère est présent dans une quantité comprise entre 0,5 % et 8 % en poids du fond de teint et dans lequel le polymère est une polyvinylpyrrolidone.

19. Procédé selon la revendication 15, dans lequel
(i) le dioxyde de titane ultrafin présente une taille moyenne des particules comprise entre environ 10 nm et environ 100 nm, et
(ii) la surface des particules du dioxyde de titane ultrafin a été traitée de façon hydrophobe pour rendre hydrophobes les particules par un agent choisi dans le groupe constitué de siloxanes, d'agents de couplage du silane, d'agents de couplage du titanate, d'agents de couplage de l'aluminium, d'agents de couplage du fluor, d'acides gras supérieurs, d'alcools supérieurs, d'amines ayant au moins un groupe alkyle comprenant au moins six atomes de carbone, et de mélanges de ceux-ci.

20. Procédé selon la revendication 19, dans lequel le dioxyde de titane ultrafin est recouvert par un oxyde ou un hydroxyde de métal qui est choisi dans le groupe constitué de l'aluminium, de la silice, du zirconium, du titane, du zinc et de l'étain.

## Patentansprüche

1. Grundierung in Form einer Wasser-in-Öl-Emulsion, wobei die wäßrige innere Phase umfaßt:
a) 0,1 bis 10 Gew.-% der Grundierung eines wasserlöslichen oder -dispergierbaren Polymeren;
b) 0,5 bis 30 Gew.-% der Grundierung eines plastifizierenden Lösungsmittels; und
c) 1 bis 30 Gew.-% der Grundierung einer ultrafeinen Titandioxid-Wasser-Dispersion, wobei die ultrafeine Titandioxid-Wasser-Dispersion 0,2 bis 18 Gew.-% der Grundierung eines ultrafeinen Titandioxids und 0,002 bis 7,2 Gew.-% der Grundierung eines nichtionischen Tensids umfaßt.

2. Grundierung nach Anspruch 1, wobei das nichtionische Tensid aus der Gruppe gewählt ist, bestehend aus Polyoxyethylensorbitanfettsäureestern, Polyoxyethylenalkylethern, Dimethiconcopolyolen, Sorbitanfettsäureestern, Zukkerfettsäureestern und Mischungen hiervon.

3. Grundierung nach Anspruch 1, wobei das nichtionische Tensid aus der Gruppe gewählt ist, bestehend aus Dimethiconcopolyolen, Polyoxyethylensorbitanfettsäureestern, und Mischungen hiervon.

4. Grundierung nach Anspruchs 1, wobei das plastifizierende Lösungsmittel aus der Gruppe gewählt ist, bestehend aus Propylenglycol, Butylenglycol, Dipropylenglycol, Glyzerin und Mischungen hiervon.

5. Grundierung nach Anspruch 1, wobei das Polymer aus der Gruppe gewählt ist, bestehend aus Sulfopolyesterharzen, wasserdispergierbaren Acrylharzen, Polyvinylacetat/Polyvinylalkohol-Harzen, Polyvinylpyrrolidon, Polyvinylpyrrolidon/Polyvinylalkohol-Copolymeren und Mischungen hiervon.

6. Grundierung nach Anspruch 1, wobei das Polymer in einer Menge von 0,5 bis 8 Gew.-% der Grundierung vorliegt.

7. Grundierung nach Anspruch 6, wobei
(i) das plastifizierende Lösungsmittel in einer Menge von 5 bis 20 Gew.-% der Grundierung vorliegt und das Lösungsmittel Propylenglycol ist, und
(ii) das Polymer in einer Menge von 0,5 bis 8 Gew.-% der Grundierung vorliegt und das Polymer Polyvinylpyrrolidon ist.

8. Grundierung nach Anspruch 6, wobei
(i) das ultrafeine Titandioxid eine durchschnittliche Teilchengröße von etwa 10 nm bis etwa 100 nm aufweist, und
(ii) die Teilchenoberfläche des ultrafeinen Titandioxids hydrophob behandelt worden ist, um die Teilchen hydrophob zu machen, durch ein Mittel, gewählt aus der Gruppe, bestehend aus Siloxanen, Silan-Kupplungsmitteln, Titanat-Kupplungsmitteln, Aluminium-Kupplungsmitteln, Fluor-Kupplungsmitteln, höheren Fettsäuren, höheren Alkoholen, Aminen mit mindestens einer Alkylgruppe mit mindestens 6 Kohlenstoffatomen und Mischungen hiervon.

9. Grundierung nach Anspruch 8, wobei das ultrafeine Titandioxid bedeckt ist durch ein Oxid oder Hydroxid eines Metalls, das aus der Aluminium, Silica, Zirkonium, Titan, Zink und Zinn umfassenden Gruppe gewählt ist.

10. Verfahren zur Herstellung einer stabilen Grundierung in Form einer Wasser-in-Öl-Emulsion, umfassend die Schritte:
(a) Vermischen öllöslicher Komponenten, um eine Ölmischung zu erhalten,
(b) Vermischen eines wasserlöslichen oder -dispergierbaren Polymeren, eines plastifizierenden Lösungsmittels und wasserlösliche Additive, um eine wäßrige Mischung zu erhalten,
(c) Vermischen der wäßrigen Mischung und der Ölmischung miteinander, um eine Emulsion zu erhalten, und
(d) Vermischen der Emulsion und einer ultrafeinen Titandioxid-Wasser-Dispersion miteinander, wobei die ultrafeine Titandioxid-Wasser-Dispersion ein ultrafeines Titandioxid und ein nichtionisches Tensid umfaßt.

11. Verfahren nach Anspruch 10, wobei die öllöslichen Komponenten ein Silikon, einen Emulgator, ein Färbemittel oder Pigment und ein Dispergiermittel umfassen.

12. Verfahren nach Anspruch 10, wobei die wasserlöslichen Additive ein Feuchthaltemittel, einen Elektrolyten und Wasser umfassen.

13. Verfahren nach Anspruch 10, wobei ein Silikon in den öllöslichen Komponenten vorliegt, und sämtliche Schritte bei einer Temperatur von 23°C bis 30°C durchgeführt werden.

14. Verfahren nach Anspruch 10, wobei das nichtionische Tensid aus der Gruppe gewählt ist, bestehend aus Dimethiconcopolyolen und Polyoxyethylensorbitanfettsäureestern.

15. Verfahren nach Anspruch 10, umfassend die Schritte
(a) Vermischen von 20 bis 80 Gew.-% der Grundierung an öllöslichen Komponenten, um eine Ölmischung zu erhalten,
(b) Vermischen von 0,1 bis 10 Gew.-% der Grundierung eines wasserlöslichen oder wasserdispergierbaren Polymeren; von 0,5 bis 30 Gew.-% der Grundierung eines plastifizierenden Lösungsmittels; und von 10 bis 75 Gew.-% der Grundierung wasserlöslicher Additive; um eine wäßrige Mischung zu erhalten,
(c) Vermischen der wäßrigen Mischung und der Ölmischung miteinander, um eine Emulsion zu erhalten, und
(d) Vermischen der Emulsion und von 1 bis 30 Gew.-% der Grundierung einer ultrafeinen Titandioxid-Wasser-Dispersion miteinander, wobei die ultrafeine Titandioxid-Wasser-Dispersion 0,2 bis 18 Gew.-% der Grundierung eines ultrafeinen Titandioxids und 0,002 bis 7,2 Gew.-% der Grundierung eines nichtionischen Tensids umfaßt.

16. Verfahren nach Anspruch 15, wobei die wasserlöslichen Additive ein Feuchthaltemittel, einen Elektrolyten und Wasser umfassen.

17. Verfahren nach Anspruch 15, wobei das Polymer aus der Gruppe gewählt ist, bestehend aus Sulfopolyesterharzen, wasserdispergierbaren Acrylharzen, Polyvinylacetat/Polyvinylalkohol-Harzen, Polyvinylpyrrolidon, Polyvinylpyrrolidol/Polyvinylalkohol-Copolymeren und Mischungen hiervon.

18. Verfahren nach Anspruch 15, wobei das Polymer in einer Menge von 0,5 bis 8 Gew.-% der Grundierung vorliegt und das Polymer Polyvinylpyrrolidon ist.

19. Verfahren nach Anspruch 15, wobei (i) das ultrafeine Titandioxid eine durchschnittliche Teilchengröße von etwa 10 nm bis etwa 100 nm aufweist, und (ii) die Teilchenoberfläche des ultrafeinen Titandioxids hydrophob behandelt worden ist, um die Teilchen hydrophob zu machen, durch ein Mittel, gewählt aus der Gruppe, bestehend aus Siloxanen, Silan-Kupplungsmitteln, Titanat-Kupplungsmitteln, Aluminium-Kupplungsmitteln, Fluor - Kupplungsmitteln, höheren Fettsäuren, höheren Alkoholen, Aminen mit mindestens einer Alkylgruppe mit mindestens 6 Kohlenstoffatomen und Mischungen hiervon.

20. Verfahren nach Anspruch 19, wobei das ultrafeine Titandioxid bedeckt ist durch ein Oxid oder Hydroxid eines Metalls, das aus der Aluminium, Silica, Zirkonium, Titan, Zink und Zinn umfassenden Gruppe gewählt ist.
